(19) **Europäisches Patentamt / European Patent Office / Office européen des brevets**

(11) **EP 4 171 690 B1**

(12) **EUROPEAN PATENT SPECIFICATION**

(45) Date of publication and mention
of the grant of the patent:
**12.03.2025 Bulletin 2025/11**

(21) Application number: **21790754.2**

(22) Date of filing: **21.09.2021**

(51) International Patent Classification (IPC):
**B05B 9/08** *(2006.01)*     **A61M 15/00** *(2006.01)*
**B05B 12/08** *(2006.01)*     **A61M 11/02** *(2006.01)*
**A61M 13/00** *(2006.01)*

(52) Cooperative Patent Classification (CPC):
**A61M 15/009; A61M 13/003;** A61M 11/02;
A61M 15/0005; A61M 15/0036; A61M 15/0041;
A61M 2202/0007; A61M 2202/0225;
A61M 2202/064; A61M 2205/8225; B05B 9/0833;
B05B 12/087

(86) International application number:
**PCT/US2021/071523**

(87) International publication number:
**WO 2022/067304 (31.03.2022 Gazette 2022/13)**

(54) **PRESSURE RELEASE FOR MEDICAL DEVICES**

DRUCKENTLASTUNG FÜR MEDIZINISCHE VORRICHTUNGEN

LIBÉRATION DE PRESSION POUR DISPOSITIFS MÉDICAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.09.2020 US 202063081659 P**

(43) Date of publication of application:
**03.05.2023 Bulletin 2023/18**

(73) Proprietor: **Boston Scientific Scimed, Inc.
Maple Grove, MN 55311 (US)**

(72) Inventors:
• **PIC, Andrew**
**Northboro, MA 01532 (US)**
• **EVERS, Ryan**
**Billerica, MA 01821 (US)**

• **MURRAY, Collin**
**Bolton, MA 01740 (US)**
• **CONGDON, Daniel**
**Hudson, MA 01749 (US)**
• **JAGELSKI, Matthew R.**
**Milford, MA 01757 (US)**

(74) Representative: **Vossius & Partner
Patentanwälte Rechtsanwälte mbB
Siebertstraße 3
81675 München (DE)**

(56) References cited:
EP-B1- 3 052 168          WO-A1-2019/118871
DE-A1- 102004 025 330     US-A- 5 399 159
US-A1- 2008 141 991       US-A1- 2020 100 986

**EP 4 171 690 B1**

**Description**

**TECHNICAL FIELD**

The disclosure relates generally to medical systems and devices for delivering pressurized fluids, and in examples, to methods and tools for releasing fluid from a medical device that includes or is connected to a pressurized fluid source.

**BACKGROUND**

Fluid delivery systems and devices are used to supply various fluids, such as a gas, during medical procedures. These procedures may include supplying fluids within a range of appropriate pressures and/or flow rates. These fluids may include agents, e.g., hemostatic agents, optimally delivered to tissue at an appropriate pressure and/or flow rate, for the particular application.

Handheld medical fluid delivery systems often require delivering a fluid from a high pressure storage tank, such as a cartridge or similar housing. Such cartridges may be loaded into a handle of the delivery system and may energize or charge the delivery system when a seal on the cartridge is pierced by a pierce pin or similar device on the delivery system. These fluids may be under high pressures and may cause injury if the medical system is not used properly. The following medical systems for dispensing agents using pressurized fluids are known in the art, namely WO 2019/118871 A1, US 2020/100986 A1, US 5 399 159 A, EP 3 052 168 B1, DE 10 2004 025330 A1, and US 2008/141991 A1. In some instances pressurized fluid may leak from the cartridges or containment devices into a sealed lumen of the handle of the medical fluid delivery systems. Over pressurization of the lumen and the handle may result in failure of the medical fluid delivery system and may result in injury to the user or the patient. The disclosure may solve one or more of these problems or other problems in the art. The scope of the disclosure, however, is defined by the attached claims and not the ability to solve a specific problem.

**SUMMARY OF THE DISCLOSURE**

According to an aspect, a device configured to deliver a pressurized fluid includes a body having an input opening for receiving the pressurized fluid and an output opening for delivering the pressurized fluid, a handle defining a lumen, wherein the lumen is configured to receive a container containing the pressurized fluid, and at least one aperture fluidly connecting the lumen to an atmosphere external of the device.

The at least one aperture may be defined by a wall of the handle.

The at least one aperture may include a plurality of apertures, and wherein the plurality of apertures may be arranged in an asymmetrical pattern on the handle.

A distal end of the handle may be configured to be connected to the body via screw threads, and wherein the at least one aperture may extend through a portion of the screw threads.

The handle may include one or more screw threads, wherein the body may include one or more screw threads, wherein the one or more apertures may be configured to be formed through the one or more screw threads of each of the handle and the body, and wherein the one or more apertures on the handle and the one or more apertures on the body may be configured to align when the handle is attached to the body.

The one or more apertures may be a single aperture disposed at a proximalmost end of the handle, and wherein the single aperture may face a proximal direction.

The device may include a plurality of crenellations and a plurality of openings defined between adjacent crenellations, and wherein the openings may be fluidly connected to the single aperture.

The plurality of crenellations may be configured to contact a proximal most end of the container when the container is attached to the device.

A sum of a cross-sectional area of each of the plurality of openings may be equal to a cross-sectional area of the single aperture.

Only proximally facing surfaces of an outermost surface of the container may contact an innermost surface of the handle when the container is connected to the device.

The at least one aperture may be configured to release the pressurized gas in a time equal to or less than 0.5 seconds.

The body may be configured to form a hole in the container, wherein the pressurized fluid may be configured to pass from the container via the hole, and wherein a diameter of the hole may be less than or equal to 1.53 mm (0.060 inches).

A cross-sectional area of a sum of each of the at least one aperture may be equal to or greater than approximately 161 mm$^2$ (0.25 square inches).

2

The device may further include the container, and, according to the invention, a membrane covering each aperture of the at least one aperture, wherein the membrane may be configured to rupture when a pressure of the pressurized fluid within the lumen exceeds a threshold.

The pressurized fluid may have a pressure of approximately 0.965 bar (14 pounds per square inch).

According to another aspect, a device configured to deliver a pressurized fluid includes a body having an input opening for receiving the pressurized fluid and an output opening for delivering the pressurized fluid, a handle defining a lumen, wherein the lumen is configured to receive a container containing the pressurized fluid, and a plurality of apertures fluidly connecting the lumen and an outer surface of the device, wherein a cross-sectional area of a sum of each of the plurality of apertures is equal to or greater than approximately 161 mm$^2$ (0.25 square inch), and wherein the pressurized fluid is configured to be released from the lumen in approximately 0.5 seconds or less.

The plurality of apertures may be arranged in an asymmetrical shape on the handle.

The handle may be connected to the body via a threaded connection, and wherein the plurality of apertures may pass through the threaded connection.

The device may further include a membrane covering each aperture of the at least one aperture, wherein the membrane may be configured to rupture when a pressure of the pressurized fluid within the lumen is equal to or greater than a threshold.

According to another aspect, a method for controlling a fluid delivery to a body of a patient includes inserting a container containing a pressurized fluid into a lumen of a handle of a fluid delivery device, the fluid delivery device defining one or more apertures fluidly connecting the lumen to an atmosphere external the fluid delivery device, and causing an opening in the container.

## BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.

FIG. 1 is a schematic of a delivery system according to an exemplary embodiment;
FIG. 2 is a side view of a handle of the delivery system of FIG. 1 according to an embodiment;
FIG. 3 is a cross-section of the handle of FIG. 2 according to an embodiment;
FIG. 4 is a cut-out view of a handle of the delivery system of FIG. 1 according to another embodiment;
FIG. 5A is a cross-section of a handle of the delivery system of FIG. 1 according to another embodiment; and
FIG. 5B is a cross-section of the handle of FIG. 5A according to an embodiment.

## DETAILED DESCRIPTION

The disclosure is described with reference to exemplary medical systems for dispensing an agent (such as a hemostatic agent) using a pressurized fluid. The devices associated with the medical systems may improve the functionality and/or the safety of the medical systems by venting pressurized fluids to atmosphere (e.g., air outside a handle and/or the medical system) if fluid from a pressurized fluid container leaks into an undesired location in the medical system. In examples, ventilation openings provided on or in the medical system may vent pressurized fluid from the lumen of the handle and may prevent over pressurization of the handle and/or the medical fluid delivery system.

Reference to any particular procedure is provided in this disclosure only for convenience and not intended to limit the disclosure. A person of ordinary skill in the art would recognize that the concepts underlying the disclosed device and application method may be utilized in any suitable procedure, medical or otherwise. The disclosure may be understood with reference to the following description and the appended drawings, wherein like elements are referred to with the same reference numerals.

Both the foregoing general description and the following detailed description are exemplary and explanatory only and are not restrictive of the features, as claimed. As used herein, the terms "comprises," "comprising," "having," "including," or other variations thereof, are intended to cover a non-exclusive inclusion such that a process, method, article, or apparatus that comprises a list of elements does not include only those elements, but may include other elements not expressly listed or inherent to such a process, method, article, or apparatus.

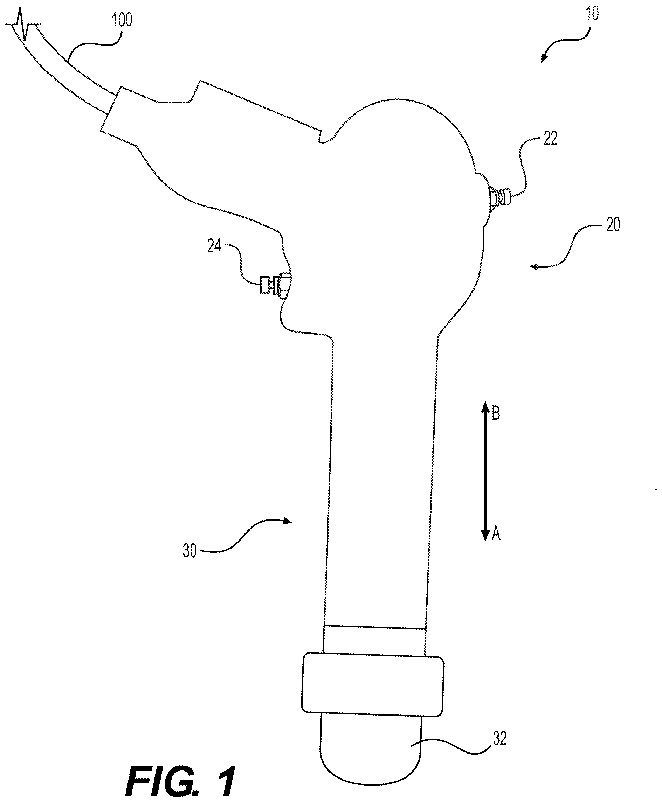

For ease of description, portions of the device and/or its components are referred to as proximal and distal portions. It should be noted that the term "proximal" is intended to refer to portions closer to a user of the device or upstream in a propellant fluid path (in the direction of arrow A in FIG. 1), and the term "distal" is used herein to refer to portions further away from the user or downstream in the propellant fluid path (in the direction of arrow B in FIG. 1). Similarly, extends "distally" indicates that a component extends in a distal direction, and extends "proximally" indicates that a component extends in a proximal direction. Further, as used herein, the terms "about," "approximately" and "substantially" indicate a range of values within +/- 10% of a stated or implied value. Additionally, terms that indicate the geometric shape of a component/surface refer only to approximate shapes.

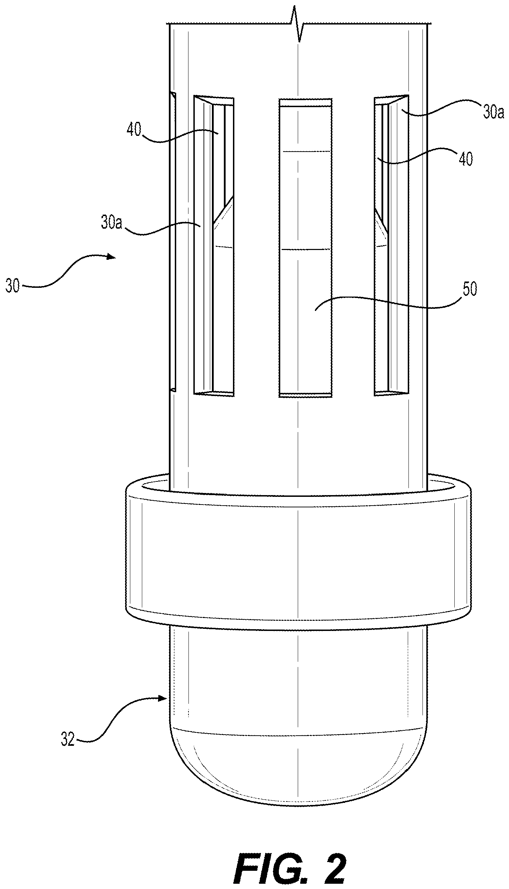

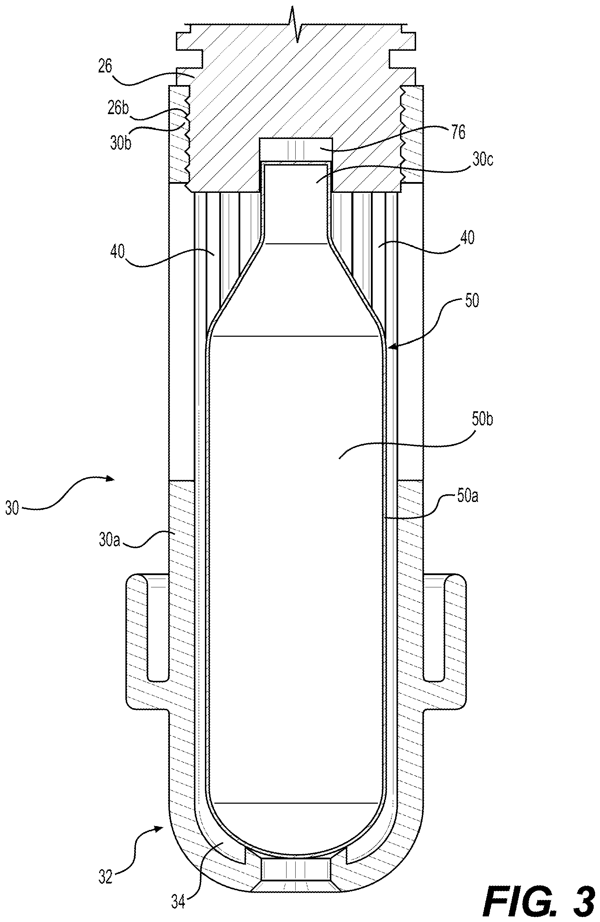

Referring to FIG. 1, a delivery system 10 according to an embodiment is shown. Delivery system 10 includes an application device 20, e.g., a hand-held device, having a handle 30 at a proximal end, and one or more triggers or actuators 22, 24 configured to actuate delivery system 10 to release a propellant fluid. A tube 100 (e.g., a catheter), or an application tip, may be attached to a distal outlet of delivery system 10 to aid in supplying the propellant fluid and/or a mixture of the propellant fluid and a hemostatic agent (or other agent) to a desired location. As will be described herein, a containment device 50 (e.g., a cartridge or container) may be contained within handle 30 (FIG. 2). A cap 32 may be releasably attached to handle 30 and may control and/or assist in the attachment of containment device 50 to application device 20 within handle 30. Cap 32 may be a proximalmost end of handle 30 in the instance that handle 30 is twisted onto delivery system 10 via threads positioned at a distal end of handle 30 (e.g., threads 30b on handle 30 and threads 26b on application device 20 shown in FIG. 3). An example of delivery system 10 is shown in U.S. Application No. 16/589,633, filed October 1, 2019.

With reference to FIGS. 2 and 3, containment device 50 is configured to contain a propellant fluid, such as a gas, e.g., carbon dioxide or any other propellant gas or fluid known in the art. While shown as a cylinder, containment device 50 may be any shape, such as a torpedo-shape, a sphere, or any other shape known in the art for containing gas. For example, containment device 50 could be a carbon dioxide cylinder for insertion into a lumen 34 of handle 30 (FIG. 3). Containment device 50 includes one or more outer walls 50a defining one or more inner chambers 50b, inner chamber(s) 50b configured to contain the propellant fluid. Walls 50a of containment device 50 may be formed of any material suitable for containing the fluid, such as but not limited to a metal alloy, a ceramic, or other material known in the art. The fluid contained in inner chamber 50b of containment device 50 may be under pressure. Accordingly, walls 50a are formed of a material and/or a thickness suitable to contain the fluid at a pressure of, for example, at least approximately 68.948 bar (1000 pounds per square inch (PSI)), or approximately 58.606 bar (850 pounds per square inch (PSI)), For example, gases which may be contained in containment device 50 include carbon dioxide ($CO_2$) having a vapor pressure of approximately 2,000-8,000 kPa at typical device temperatures, or nitrogen ($N_2$) having a vapor pressure less than 40 MPa at typical device temperatures. It will be understood that these gases are examples and are not limiting to the types of gases contained in containment device 50.

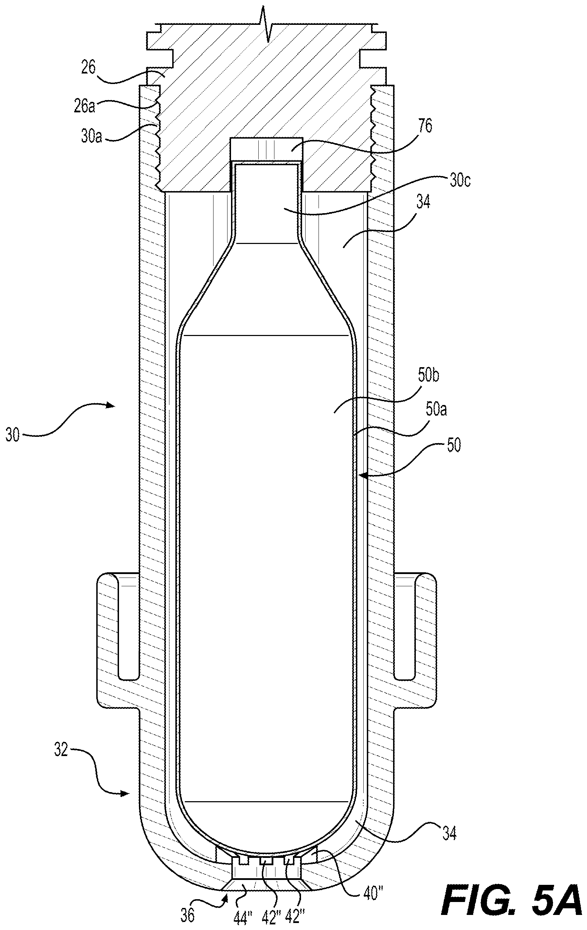

With continued reference to FIG. 3, application device 20 is attached to containment device 50 by inserting containment device 50 into lumen 34 of handle 30. For example, an inlet (e.g., inlet 76 in FIGS. 3 and 5A) of application device 20 may be connected directly to an output, such as a protuberance 30c (FIGS. 3, 4, and 5A) of containment device 50 using a threaded connection, pressure washer adapter, or the like. Protuberance 30c of containment device 50 may extend into inlet 76 of application device 20 and connect, directly or indirectly, to a regulator (not shown). Directly connecting application device 20 to containment device 50 may be suitable for, e.g., a small-volume containment device 20 containing approximately 5 g to 75 g of compressed gas, or preferably approximately 12 g to 40 g of compressed gas, to allow for greater portability of delivery system 10.

Referring to FIG. 1, actuation of one or both of actuating devices 22, 24 of application device 20 causes the fluid to exit delivery system 10 to a target site via tube 100. It will be understood that only one actuating device 22, 24 may need to be actuated in some embodiments. Actuation of one or both actuating devices 22, 24 releases a buildup of pressure within delivery system 10, causing the regulator to release fluid from containment device 50 at a predetermined pressure to delivery system 10 downstream of the regulator. Application device 20 may be, e.g., a garden-hose handle or other pistol-like configuration. Actuating devices 22, 24 may be any push button, trigger mechanism, or other device that, when actuated, opens a valve and releases fluid, as will be described in greater detail herein.

Cap 32 may be attached to a proximal end of handle 30, or handle 30 may be attached to a proximal end of application device 20 (see FIG. 3) such that lumen 34 of handle 30 is fluidly sealed (absent the apertures described herein). Handle 30 may be attached to the proximal end of application device 20 using a threaded connection (e.g., threads 30b and 26b in FIG. 3). For example, after containment device 50 is inserted into lumen 34, handle 30 may be placed at the proximal end of application device 20. An inner surface of a wall 30a at a proximal end of handle 30 may contact containment device 50. Handle 30 may be twisted onto application device 20 via the threaded connection, e.g., threads 30b of handle 30 and threads 26b of application device 20. In some instances, as handle 30 is twisted onto application device 20, the proximal end of wall 30a may urge containment device 50 toward a pierce pin located at inlet 76 of application device 20, such that a distal end 30c of containment device 50 moves into fluid communication with a fluid path of application device 20. Alternatively, containment device 50 may be urged toward the pierce pin by cap 32 when cap 32 is attached to the proximal end of handle 30, as in U.S. Application No. 16/589,633, filed October 1, 2019.

In this manner, containment device 50 may be in fluid connection with the fluid path of application device 20, and the fluid propellant may be used to supply an agent from application device 20 to a target site via tube 100. As yet another example, a lever (not shown) may be used to urge containment device 50 in the distal direction to bring containment device 50 into fluid communication with the fluid path of application device 20.

However, sealing containment device 50 within lumen 34 of handle 30 may cause fluid pressure to increase if containment device 50 is not properly connected to application device 20 (e.g., a leak in a seal, eroded threads, etc.) and/or if containment device 50 is damaged and leaks pressurized fluid into lumen 34. Thus, there may be a need to vent fluid from lumen 34 to atmosphere (e.g., outside handle 30).

With reference to FIGS. 2 and 3, apertures 40 are formed in sidewall 30a of handle 30. Apertures 40 fluidly connect an outer surface of handle 30, e.g., an atmosphere or a neutral atmosphere, to lumen 34 of handle 30. Apertures 40 are rectangular in FIG. 2, but are not limited to this shape. For example, apertures 40 may be circular, oval, triangular, curved, spiral, sinusoidal, or irregular. Apertures 40 may extend along an entire length of handle 30 from the proximal end to the distal end, or apertures 40 may be disposed on only a portion of handle 30. Alternatively, apertures 40 may be placed asymmetrically about handle 30 based on ergonomics. For example, apertures 40 may be placed in areas of handle 30 where a user is less likely to grasp handle 30 during use. In this manner, apertures 40 may not be covered by a user's hand during use.

Apertures 40 may have a cross-sectional area sufficient to allow a fluid to pass from lumen 34 to the neutral atmosphere in the event fluid leaks from containment device 50 and does not pass into the fluid pathway of delivery system 10. According to an example, apertures 40 may have a cross-sectional area large enough to permit fluid to escape within approximately 0.5 seconds. For example, once fluid is released into lumen 34 from containment device 50, the fluid may be dispersed from lumen 34 within 0.5 seconds. For example, in the event containment device 50 is not properly attached to the fluid pathway of delivery system 10, or in the event wall 50a of containment device 50 fails and pressurized fluid is released into lumen 34 of handle 30, the pressurized fluid may be vented to atmosphere to prevent pressure buildup in handle 30. In the absence of apertures 40, pressure buildup within lumen 34 of handle 30 may cause wall 30a of handle 30 to burst, which may cause injury to the user and/or the patient.

For example, if the pressure of the fluid in lumen 34 is at room temperature (25 degrees C) at an average pressure of 58.606 bar (850 pounds per square inch (PSI)), and an opening in containment device 50 is approximately 1.53 mm (0.060 inches) in diameter, sidewall 30a of handle 30 may rupture if the fluid is not vented within approximately 0.5 seconds. According to an example, a total cross-sectional area of all ventilation openings may be greater than approximately 161 mm$^2$ (0.25 square inches). In other words, a sum of the cross-sectional areas of each aperture 40 in handle 30 may be greater than approximately 161 mm2 (0.25 square inches). This cross-sectional area of apertures 40 may provide a release of pressurized fluid equal to or less than approximately 0.5 seconds.

As one example, in the event aperture 40 is a single cylinder (extending through sidewall 30a from lumen 34 to atmosphere) having a circular cross-section, a minimum radius of the circular cross-section of aperture 40 may be determined based on Formula 1. In Formula 1, R is the radius of the circular cross-section of aperture 40, Q is the flow rate of the fluid through aperture 40 in standard liters per minute (Q is predetermined value based at least in part on the time in which lumen 34 is to be vented, e.g., 0.5 seconds), $\eta$ is the viscosity of the fluid flowing through aperture 40, L is a thickness of handle 30 (e.g., a length of aperture 40 from lumen 34 to the atmosphere), and $\Delta P$ is a pressure difference (in bar or PSI) between a pressure inside lumen 34 and atmospheric pressure.

Formula 1:

$$R \geq \sqrt[4]{\frac{8Q\eta L}{\Delta P \Pi}}$$

In some examples, a membrane (not shown) may cover each aperture 40, or a burst disc or pressure relief valve may be used in place of one or more apertures 40. The membrane may be provided on an outer surface and/or an inner surface of handle 30, may be any color, and may be translucent or transparent. A material of the membrane may include preformed separation zones and/or or perforations to assist in rupturing at a threshold. The membrane may be made of a flexible material and/or a material suitable to rupture when a pressure within lumen 34 exceeds a threshold, e.g., greater than or equal to approximately 0.965 bar (14 pounds per square inch (PSI)). In some examples, the pressure may change based on atmospheric pressure outside handle 30 (e.g., atmospheric pressure may change based on a location of use of the device). Similarly, a burst disc or pressure relief valve may open once the pressure with lumen 34 exceeds a threshold.

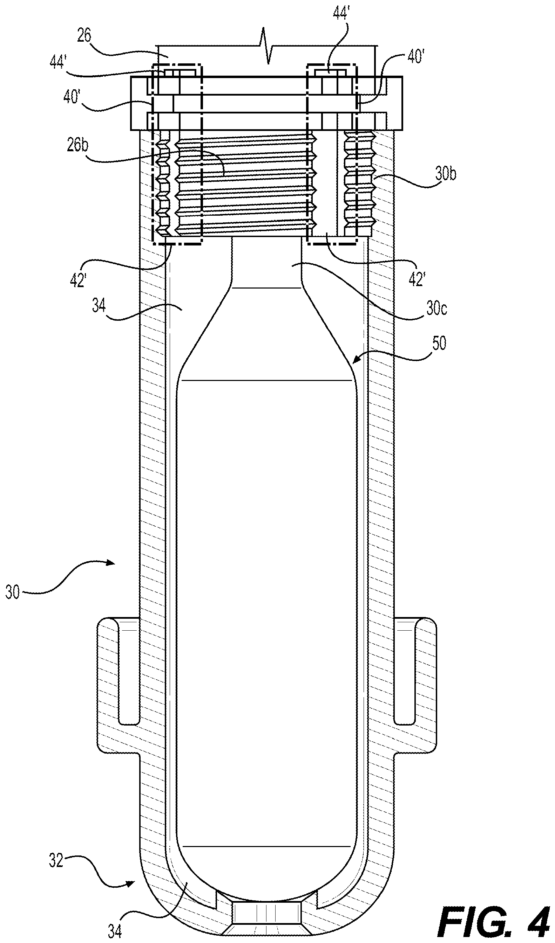

Apertures 40' according to another example are shown in FIG. 4. As discussed herein, containment device 50 may be attached to application device 20 via threads 30b on handle 30 and threads 26b on application device 20. One or more apertures 40' may be formed through and 26b. For example, apertures 40' may extend from an opening 42' at a proximalmost end of threads 26b to an opening 44' at a distalmost end of threads 26b. Opening 44' is open to and/or in fluid communication with the external atmosphere. In some instances, apertures 40' may be formed by removing a portion of threads 26b at locations around a circumference of the threaded portion of application device 20. That portion may be part of a regulator or pierce system. For example, threads 26b may be disconnected at a same circumferential location along an entire length of threads 26b to form apertures 40', as shown in FIG. 4. Alternatively, or additionally, apertures 40' may be formed in threads 30b of handle 30. In some instances, apertures 40' formed on handle 30 may match up with apertures 40' on application device 20 when handle 30 is completely attached to application device 20. This may increase the cross-

**EP 4 171 690 B1**

sectional area of apertures 40', which may increase the ventilation of pressurized fluid from lumen 34. In some cases, a membrane may cover one or both of openings 42', 44' of apertures 40'. The membrane may be made of a flexible material and/or a material suitable to rupture when a pressure within lumen 34 exceeds a threshold, such as the pressures mentioned above.

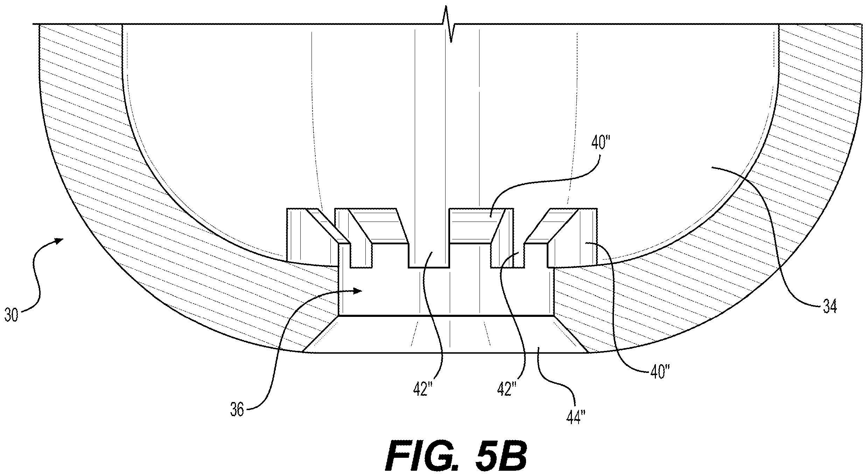

Another example of a fluid release mechanism is shown with reference to FIGS. 5A and 5B. Handle 30 includes a member 36 at the proximal most end of handle 30. Member 36 includes crenellations 40" (supports), and a plurality of apertures 42" defined between adjacent crenellations 40". In some instances, a portion or entirety of member 36 may include a material that increases a friction coefficient between member 36 and containment device 50 to assist in urging containment device 50 in the distal direction. As discussed herein, containment device 50 may be urged toward the pierce pin by an inner surface of handle 30 (e.g., cap 32 or the proximal end of handle 30) as handle 30 is twisted onto application device 20, or as cap 32 is twisted onto application device 20. In this situation, crenellations 40" may contact a proximalmost outer surface of containment device 50 and support device 50 above apertures 42". As handle 30 and/or cap 32 are moved toward application device 20 via a twisting motion, crenellations 40" may contact the proximal most outer surface of containment device 50 to aid in the movement of containment device 50 in the distal direction (e.g., in the direction indicated by arrow B in FIG. 1). Crenellations 40" may assist in moving containment device in the distal direction, and/or may aid in creating a space between an outermost surface of containment device 50 and an innermost surface of sidewall 30a. This space (e.g., a portion of lumen 34) may allow any fluid escaping from containment device 50 to pass unimpeded to apertures 42" and to an outside atmosphere, as discussed herein. It will be understood that any number of crenellations 42" may be formed, for example, two, three, or more crenellations 42".

A proximal-facing opening 44" may be formed in member 36. Opening 44" is fluidly connected to each of apertures 42", which are in turn fluidly connected to lumen 34 of handle 30. When handle 30 is connected to application device 20, apertures 42" and opening 44" allow any pressurized fluid buildup within lumen 34 to be vented to an atmosphere. In an example, venting may occur in approximately 0.5 seconds. For example, as discussed herein, an opening or an aperture may have a size of approximately 161.3 mm$^2$ (0.25 square inches). or greater to allow any fluid buildup to pass to the outside atmosphere. In this case, the sum of the cross-sectional areas of all apertures 42" may be greater than or equal to 161.3 mm$^2$ (0.25 square inches). and the cross-sectional area of opening 44" may be greater than or equal to approximately 161.3 mm$^2$ (0.25 square inches).

It will also be understood that any pathway formed between apertures 42" and opening 44" (e.g., a sidewall of member 36) may have a cross-sectional area of greater than or equal to approximately 161.3 mm$^2$ (0.25 square inches). It will be understood that member 36 may be integral with handle 30, and not a separate part or component. For example, crenellations 40" may be formed integrally with handle 30, e.g., integrally molded, leaving apertures 42" and opening 44" in the molding process.

While the apertures described herein have been described as having a total cross-sectional area of greater than or equal to approximately 161.3 mm$^2$ (0.25 square inches), it will be understood that this is an example and may change based on design factors. For example, pressurized fluid may be dispersed from lumen 34 in less than or equal to approximately 0.5 seconds. If the fluid is not dispersed from lumen 34 in this timeframe or other suitable timeframe depending on design factors (including the strength of sidewall 30a), the structural integrity of sidewall 30a may fail, causing injury. In some instances, containment device 50 may include a pressurized fluid greater than 58.606 bar (850 pounds per square inch (PSI)), and/or delivery system 10 may be designed for use at a temperature different from room temperature (e.g., approximately 25 degrees C). In these instances, the apertures and openings described herein may be designed to have a cross-sectional area large enough to disperse the pressurized fluid in less than or equal to approximately 0.5 seconds, or other suitable timeframe, based on Formula 1 and the corresponding discussion above. It will also be understood that one or more of apertures 40, 40', and/or 42" may be combined in a same delivery system 10 to provide fluid dispersement.

It will be apparent to those skilled in the art that various modifications and variations can be made to the disclosed device without departing from the scope of the disclosure. Other embodiments of the disclosure will be apparent to those skilled in the art from consideration of the specification and practice of the invention disclosed herein. It is intended that the specification and examples be considered as exemplary only, with a true scope of the invention being indicated by the following claims.

**Claims**

**1.** A medical device (20) configured to dispense a hemostatic agent using a pressurized fluid, the device comprising:

a body having an input opening for receiving the pressurized fluid and an output opening for delivering the pressurized fluid;
a handle (30) defining a lumen (34), wherein the lumen (34) is configured to receive a container containing the pressurized fluid; and

at least one aperture (40) fluidly connecting the lumen (34) to an atmosphere external of the device and configured to vent fluid from the lumen (34) to the atmosphere external of the device, and

wherein the at least one aperture (40) is formed in a sidewall (30a) of the handle (30),

**characterized in that** a membrane covering each aperture (40) of the at least one aperture (40), and wherein the membrane is configured to rupture when a pressure of the pressurized fluid within the lumen (34) exceeds a threshold.

2. The device according to claim 1, wherein the at least one aperture (40) includes a plurality of apertures (40), and wherein the plurality of apertures (40) are arranged in an asymmetrical pattern on the handle (30).

3. The device according to claim 1, wherein a distal end (30c) of the handle (30) is configured to be connected to the body via screw threads, and wherein the at least one aperture (40) extends through a portion of the screw threads.

4. The device according to claim 3, wherein the handle (30) includes one or more screw threads, wherein the body includes one or more screw threads, wherein the one or more apertures (40) are configured to be formed through the one or more screw threads of each of the handle (30) and the body, and wherein the one or more apertures (40) on the handle (30) and the one or more apertures (40) on the body are configured to align when the handle (30) is attached to the body.

5. The device according to any of the preceding claims, wherein only proximally facing surfaces of an outermost surface of the container contacts an innermost surface of the handle (30) when the container is connected to the device.

6. The device according to any of the preceding claims, comprising the container containing the pressurized fluid and wherein the at least one aperture (40) is configured to release the pressurized gas in a time equal to or less than 0.5 seconds.

7. The device according to any of the preceding claims, wherein the body is configured to form a hole in the container, wherein the pressurized fluid is configured to pass from the container via the hole, and wherein a diameter of the hole is less than or equal to 1.53 mm (0.060 inches).

8. The device according to any of the preceding claims, wherein the pressurized fluid has a pressure of approximately 0.965 bar (14 pounds per square inch).

9. The device according to any of the preceding claims, wherein the at least one aperture is placed in areas of the handle (30), where a user is less likely to grasp the handle (30) during use such that the at least one aperture (40) is not covered by a user's hand during use.

**Patentansprüche**

1. Medizinische Vorrichtung (20), die zum Abgeben eines hämostatischen Mittels unter Verwendung eines unter Druck stehenden Fluids konfiguriert ist, wobei die Vorrichtung aufweist:

einen Körper mit einer Eingangsöffnung zum Aufnehmen des unter Druck stehenden Fluids und einer Ausgangsöffnung zum Abgeben des unter Druck stehenden Fluids;

einen Griff (30), der ein Lumen (34) definiert, wobei das Lumen (34) zum Aufnehmen eines Behälters, der das unter Druck stehende Fluid enthält, konfiguriert ist; und

mindestens eine Öffnung (40), die das Lumen (34) mit einer Atmosphäre außerhalb der Vorrichtung fluidverbindet und zum Ableiten von Fluid aus dem Lumen (34) in die Atmosphäre außerhalb der Vorrichtung konfiguriert ist, und

wobei die mindestens eine Öffnung (40) in einer Seitenwand (30a) des Griffs (30) ausgebildet ist,

**dadurch gekennzeichnet, dass** eine Membran jede Öffnung (40) der mindestens einen Öffnung (40) abdeckt, und wobei die Membran zum Reißen konfiguriert ist, wenn ein Druck des unter Druck stehenden Fluids in dem Lumen (34) einen Schwellenwert überschreitet.

2. Vorrichtung nach Anspruch 1, wobei die mindestens eine Öffnung (40) mehrere Öffnungen (40) umfasst, und wobei die mehreren Öffnungen (40) in einem asymmetrischen Muster auf dem Griff (30) angeordnet sind.

3. Vorrichtung nach Anspruch 1, wobei ein distales Ende (30c) des Griffs (30) dazu konfiguriert ist, über ein Schraubgewinde mit dem Körper verbunden zu werden, und wobei sich die mindestens eine Öffnung (40) durch einen Teil des Schraubgewindes erstreckt.

4. Vorrichtung nach Anspruch 3, wobei der Griff (30) ein oder mehrere Schraubgewinde aufweist, wobei der Körper ein oder mehrere Schraubgewinde aufweist, wobei die eine oder mehreren Öffnungen (40) so konfiguriert sind, dass sie durch das eine oder mehreren Schraubgewinde sowohl des Griffs (30) als auch des Körpers gebildet werden, und wobei die eine oder mehreren Öffnungen (40) am Griff (30) und die eine oder mehreren Öffnungen (40) am Körper so konfiguriert sind, dass sie aufeinander ausgerichtet sind , wenn der Griff (30) am Körper befestigt ist.

5. Vorrichtung nach einem der vorstehenden Ansprüche, bei der nur nach proximal weisende Flächen einer äußersten Fläche des Behälters eine innerste Fläche des Griffs (30) berühren, wenn der Behälter mit der Vorrichtung verbunden ist.

6. Vorrichtung nach einem der vorstehenden Ansprüche, die den Behälter aufweist, der das unter Druck stehende Fluid enthält, und wobei die mindestens eine Öffnung (40) zum Freisetzen des unter Druck stehenden Gases in einer Zeit von 0,5 Sekunden oder weniger konfiguriert ist.

7. Vorrichtung nach einem der vorstehenden Ansprüche, wobei der Körper so konfiguriert ist, dass er ein Loch in dem Behälter bildet, wobei das unter Druck stehende Fluid zum Fließen aus dem Behälter durch das Loch konfiguriert ist, und wobei ein Durchmesser des Lochs kleiner oder gleich 1,53 mm (0,060 Zoll) ist.

8. Vorrichtung nach einem der vorstehenden Ansprüche, wobei das unter Druck stehende Fluid einen Druck von etwa 0,965 bar (14 Pfund pro Quadratzoll) aufweist.

9. Vorrichtung nach einem der vorstehenden Ansprüche, wobei die mindestens eine Öffnung in Bereichen des Griffs (30) angeordnet ist, in denen es weniger wahrscheinlich ist, dass ein Benutzer den Griff (30) während der Benutzung ergreift, sodass die mindestens eine Öffnung (40) während der Benutzung nicht von der Hand eines Benutzers verdeckt wird.

## Revendications

1. Dispositif médical (20) configuré pour distribuer un agent hémostatique en utilisant un fluide sous pression, le dispositif comprenant :

   un corps ayant une ouverture d'entrée pour recevoir le fluide sous pression et une ouverture de sortie pour distribuer le fluide sous pression ;
   une poignée (30) définissant une lumière (34), dans lequel la lumière (34) est configurée pour recevoir un récipient contenant le fluide sous pression ; et
   au moins une ouverture (40) raccordant de manière fluidique la lumière (34) à une atmosphère extérieure du dispositif et configurée pour évacuer le fluide de la lumière (34) à l'atmosphère extérieure du dispositif, et dans lequel la au moins une ouverture (40) est formée dans une paroi latérale (30a) de la poignée (30), **caractérisé en ce qu'**une membrane recouvre chaque ouverture (40) de la au moins une ouverture (40), et dans lequel la membrane est configurée pour se rompre lorsqu'une pression du fluide sous pression dans la lumière (34) dépasse un seuil.

2. Dispositif selon la revendication 1, dans lequel la au moins une ouverture (40) comprend une pluralité d'ouvertures (40), et dans lequel la pluralité d'ouvertures (40) est agencée selon un motif asymétrique sur la poignée (30).

3. Dispositif selon la revendication 1, dans lequel une extrémité distale (30c) de la poignée (30) est configurée pour être raccordée au corps via des filetages de vis, et dans lequel la au moins une ouverture (40) s'étend à travers une partie des filetages de vis.

4. Dispositif selon la revendication 3, dans lequel la poignée (30) comprend un ou plusieurs filetages de vis, dans lequel le corps comprend un ou plusieurs filetages de vis, dans lequel les une ou plusieurs ouvertures (40) sont configurées pour être formées par le biais des un ou plusieurs filetages de vis de chacun parmi la poignée (30) et le corps, et dans lequel les une ou plusieurs ouvertures (40) sur la poignée (30) et les une ou plusieurs ouvertures (40) sur le corps sont configurées pour s'aligner lorsque la poignée (30) est fixée au corps.

5. Dispositif selon l'une quelconque des revendications précédentes, dans lequel des surfaces orientées uniquement de manière proximale de la surface située le plus à l'extérieur du récipient est en contact avec la surface située le plus à l'intérieur de la poignée (30), lorsque le récipient est raccordé au dispositif.

6. Dispositif selon l'une quelconque des revendications précédentes, comprenant le récipient contenant le fluide sous pression et dans lequel la au moins une ouverture (40) est configurée pour libérer le gaz sous pression à un temps égal ou inférieur à 0,5 seconde.

7. Dispositif selon l'une quelconque des revendications précédentes, dans lequel le corps est configuré pour former un trou dans le récipient, dans lequel le fluide sous pression est configuré pour passer du récipient via le trou, et dans lequel un diamètre du trou est inférieur ou égal à 1,53 mm (0,060 pouce).

8. Dispositif selon l'une quelconque des revendications précédentes, dans lequel le fluide sous pression a une pression d'approximativement 0,965 bar (14 livres par pouce carré).

9. Dispositif selon l'une quelconque des revendications précédentes, dans lequel la au moins une ouverture est placée dans des zones de la poignée (30), dans lesquelles un utilisateur est moins susceptible de saisir la poignée (30) pendant l'utilisation, de sorte que la au moins une ouverture (40) n'est pas recouverte par la main d'un utilisateur pendant l'utilisation.

**FIG. 1**

**FIG. 2**

*FIG. 3*

**FIG. 4**

**FIG. 5A**

**FIG. 5B**

EP 4 171 690 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019118871 A1
- US 2020100986 A1
- US 5399159 A
- EP 3052168 B1
- DE 102004025330 A1
- US 2008141991 A1
- US 58963319